# EUROPEAN PATENT APPLICATION

(11) **EP 2 737 882 A1**
(43) Date of publication of application: **04.06.2014**
(21) Application number: 12820606.7
(22) Date of filing: 07.06.2012
(51) Int. Cl.: A61F 5/451, A61F 5/453

(54) **URINE ABSORPTION DEVICE**

(30) Priority: 29.07.2011 JP 2011167811
(71) Applicant: Unicharm Corporation, Shikokuchuo-shi, Ehime 799-0111 (JP)
(72) Inventor: SUZUKI, Miou, Kanonji-shi, Kagawa 769-1602 (JP); TANIMOTO, Kei, Kanonji-shi, Kagawa 769-1602 (JP); ENDO, Hiroko, Kanonji-shi, Kagawa 769-1602 (JP)
(74) Representative: Eke, Philippa Dianne
(86) International application number: PCT/JP2012/064661
(87) International publication number: WO 2013/018435

(57) **Abstract**

A urine disposal device adapted to prevent a connector from being soiled with excrement. A urine disposal device (10) has a longitudinal direction (Y), a transverse direction (X) and a thickness direction (Z) and includes a urine receptacle (11) defining therein a urine receiving space (S) and connector (12) adapted to connect the urine receptacle (11) through a connector (12) to external equipment (O) at least including a vacuum pump (15) for suction of urine from the urine receiving space (S). The urine receptacle (11) has a first surface (G) facing a wearer' s skin, a second surface F not facing the wearer's skin when the wearer is in a supine posture and an external outlet port (connecting region) (65b) in which the connector is connected. The external outlet port (connecting region) (65b) is arranged on the second surface (F).

## Description

### {Technical Field}

The present invention relates to urine disposal devices each including a urine receptacle having a urine receiving space defined therein and external equipment at least including a vacuum pump adapted to suction such urine from the urine receiving space.

### {Background}

In the specification of the patent application No. 2011-011411, a urine disposal device is disclosed by the applicant of the present application. This urine disposal device includes a urine receptacle adapted to be put on a penis (hereinafter designated as genitalia), external equipment at least including a vacuum pump to suction urine from a receiving space and a connector serving to connect the urine disposal device to the external equipment.

In this urine disposal device, the connector at least includes a first connector tube and a joint. The first connector tube has one end joined to the vacuum pump.

The urine receptacle is disposed with a urine collecting container. The urine collecting container has an internal outlet port provided in an internal space of the container, an external outlet port (connecting region) provided on the outside of the internal space and a tubular member to put the internal outlet port and the external outlet port into a fluid-communication relationship.

Another end of the first connector tube is connected to the external outlet port and whereby the urine receptacle is connected to the vacuum pump.

### {Citation List}

### {Patent Literature}

{PTL 1}: Specification of Patent Application No. 2011-011411 {Summary}

### {Technical Problem}

Wearers of such type of urine receptacle are often the aged and/or severely disabled persons and many of these persons spend most of the day on their beds. Furthermore, these persons spend most of the time on the bed in a supine posture or in a lying posture.

In this known urine disposal device, the urine collecting container is arranged on the skin-contact surface of the urine receptacle wearer being in a supine posture and, when the wearer has evacuated the bowels (excrement), the end portion of the first connector tube will be soiled with the excrement. Thereafter, when the used urine receptacle is exchanged with the fresh one, the end portion of the first connector tube soiled with the excrement must be cleaned. Thus the known urine disposal device has left behind a problem that it takes a lot of trouble to exchange the urine receptacle.

In view of this, an object of the present invention is to provide a urine disposal device improved so as to alleviate a possibility that the connector might be soiled with the wearer's excrement.

### {Solution to Problem}

Some embodiments of the present invention provide a urine disposal device having a longitudinal direction, a transverse direction and a thickness direction and including a urine receptacle and adapted to connect the urine receptacle through a connector to external equipment at least including a vacuum pump to suction urine from the urine receptacle.

The present invention lies in that the urine receptacle has a first surface facing a wearer's skin, a second surface not facing the wearer's skin when the wearer is in a supine posture and a connecting region in which the connector is connected wherein the connecting region is arranged on the second surface.

The present invention may at least include embodiments as described below.
(1) The urine receptacle is disposed with a urine collecting container having an internal space. The urine collecting container includes an internal outlet port arranged within the internal space and an external outlet port arranged on the outside of the internal space and a tubular member adapted to put the internal outlet port and the external outlet port in a fluid-communication relationship wherein the external outlet port functions also as a connecting region.
(2) The urine receptacle has an opening extending in the transverse direction. The urine collecting container is arranged so that a urine evacuation direction from the internal outlet port to the external outlet port may be oriented oppositely to the opening.
(3) The urine receptacle has an opening extending in the transverse direction. The urine collecting container is arranged so that the urine evacuation direction from the internal outlet port to the external outlet port may be oriented towards the opening.
(4) The urine receptacle is formed from a plurality of sheets made of a thermoplastic synthetic resin in a bag-like shape defining therein a urine receiving space in fluid-communication with the opening. High stiffness regions are formed in the course of heat sealing a plurality of sheets but the high stiffness regions are formed only on lateral edges of these sheets as a portion of the opening's periphery.
(5) The respective high stiffness regions have centers. The urine collecting container is arranged so that the internal space may be positioned within an overlapping region in which a region defined under a first imaginary line extending so as to pass through the center of one of two high stiffness regions spaced apart from each other in the transverse direction and to be tangent to the internal space on the upper side of the urine collecting container and a region defined under a second imaginary line extending so as to pass through the center of the other high stiffness region and to be tangent to the internal space on the upper side of the urine collecting container overlap each other.

### {Advantageous Effects of Invention}

In the urine disposal device according to the present invention put on the wearer in a supine posture, the connecting region of the urine receptacle is arranged on the second surface not facing the wearer's skin. In consequence, when the wearer discharges excrement, the urine receptacle covers the distal end portion of the connector and the connecting region, whereby the distal end portion of the connector and the connecting region are unlikely to be soiled with the excrement. In addition, the connecting region should not come in contact with the wearer's body since the connecting region is arranged on the second surface. In this way, it is possible to alleviate a sense of discomfort felt by the wearer, even if the urine collecting container is formed of a thermoplastic synthetic resin.

### {Brief Description of Drawings}

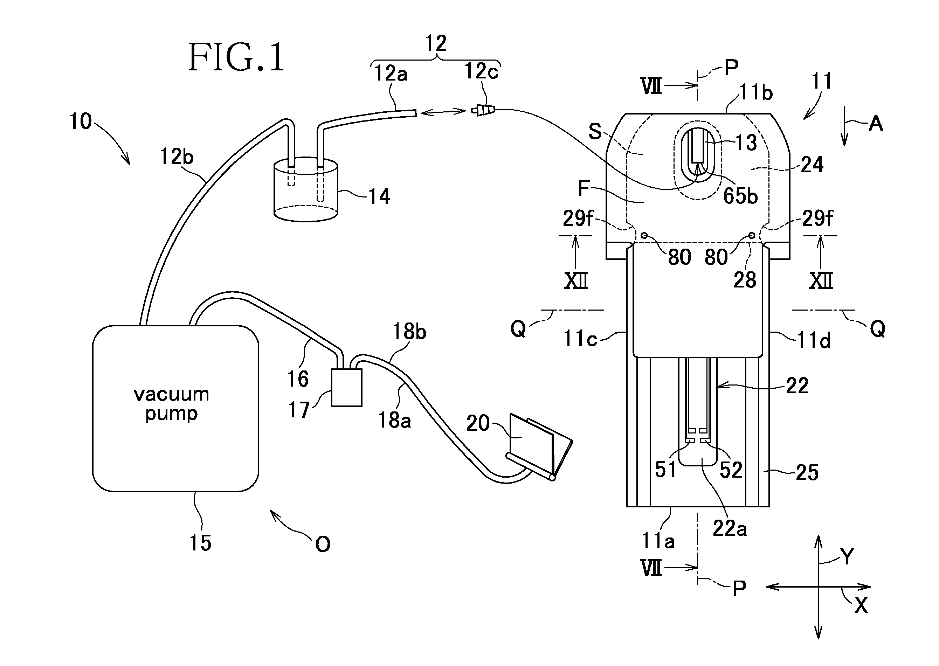

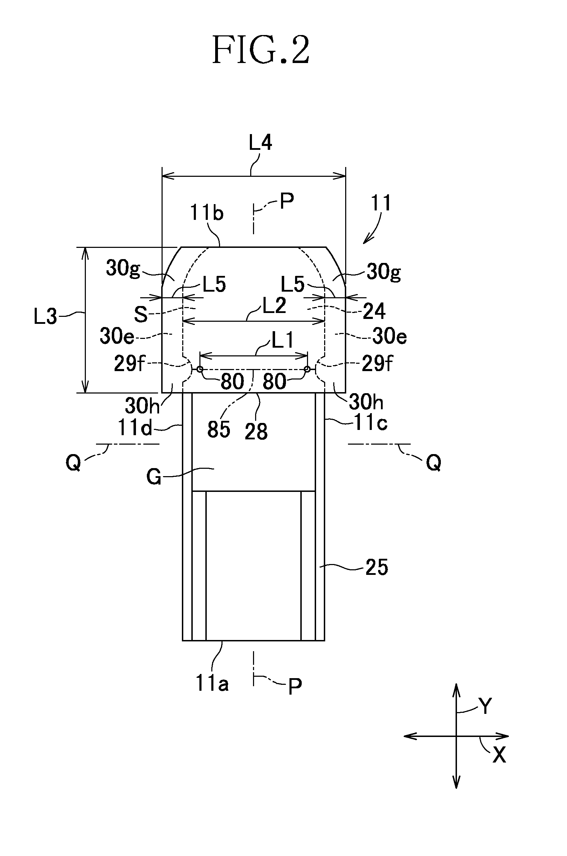

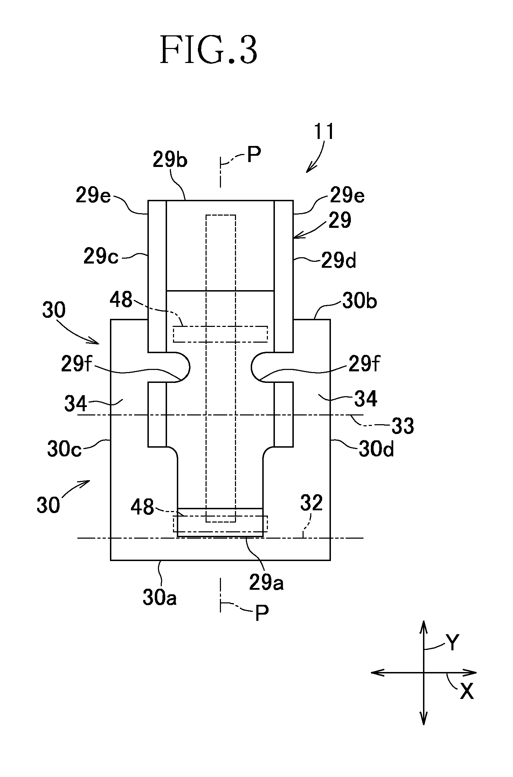

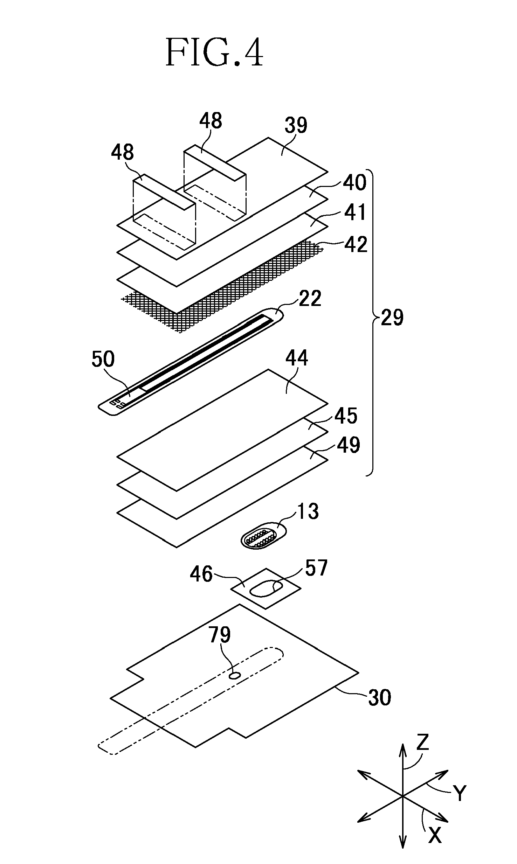

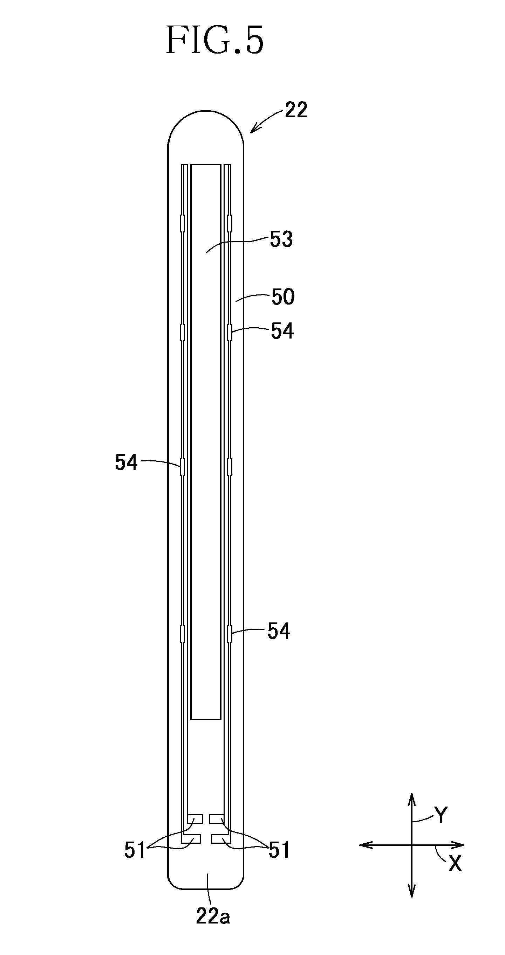

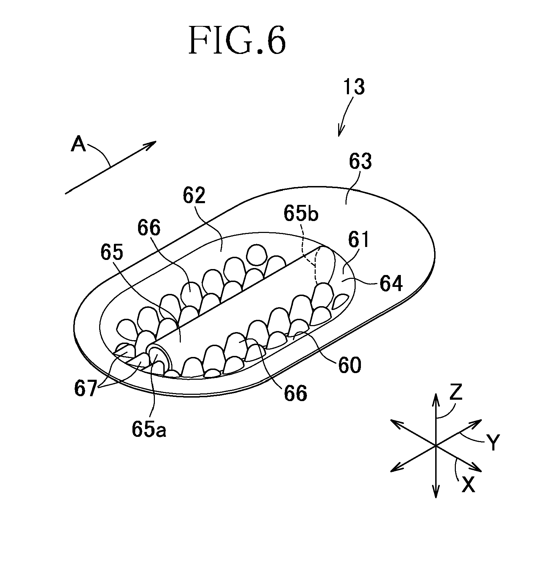

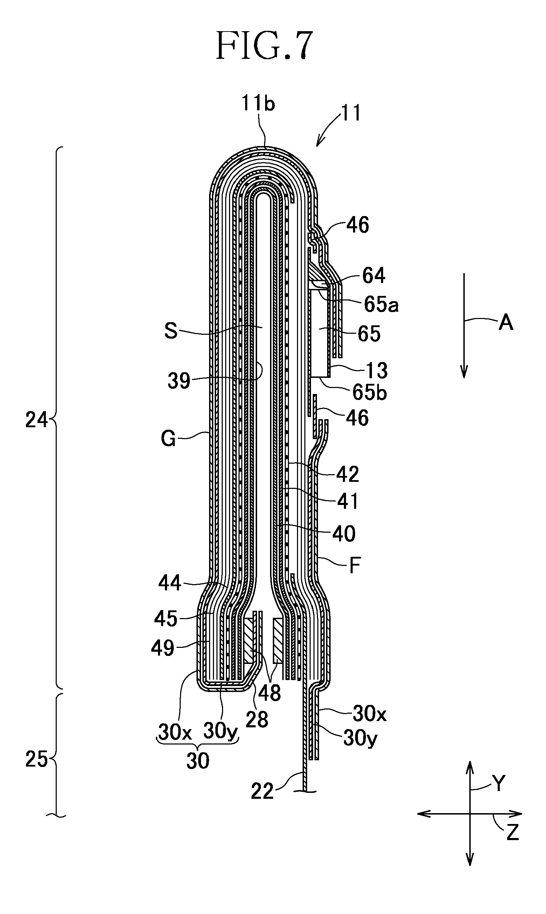

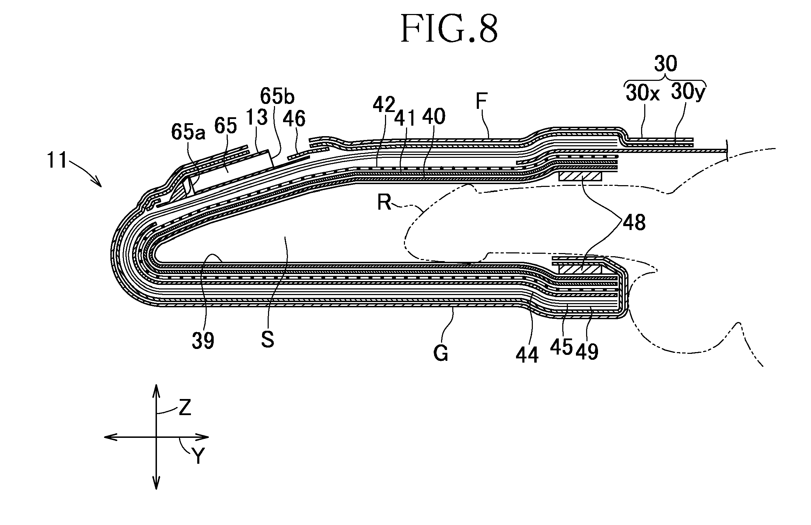

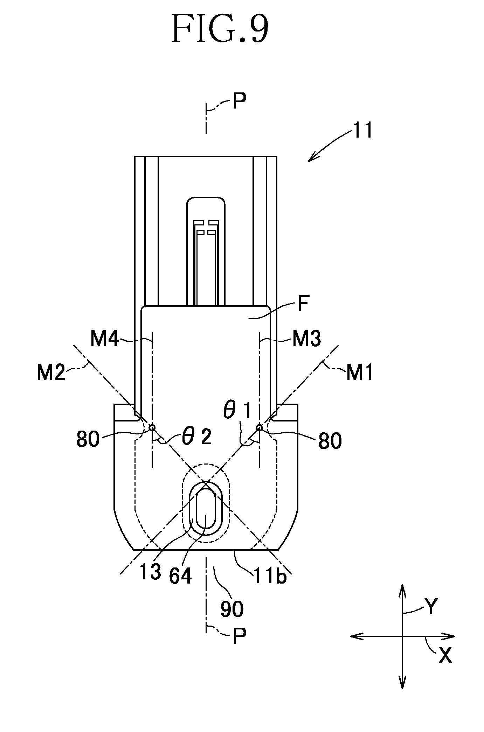

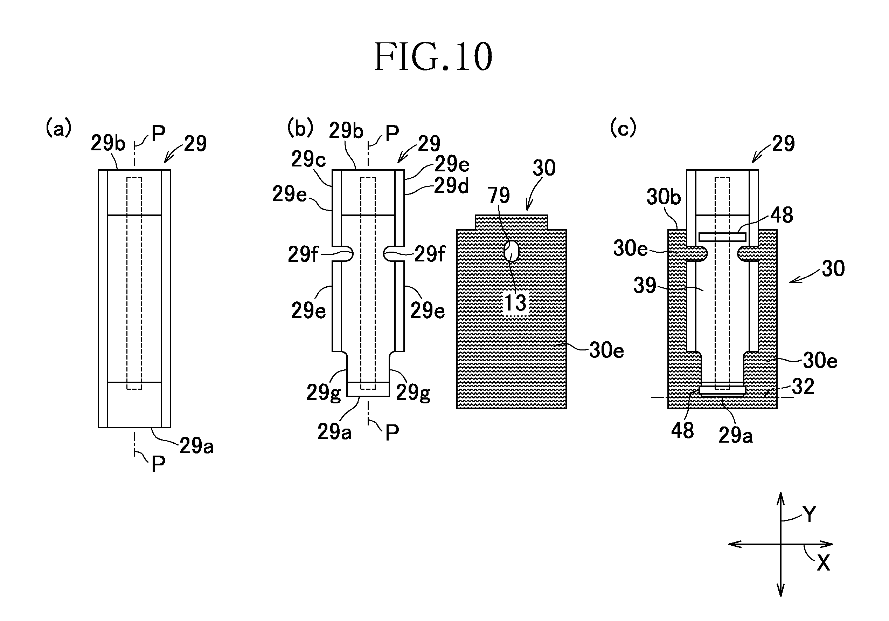

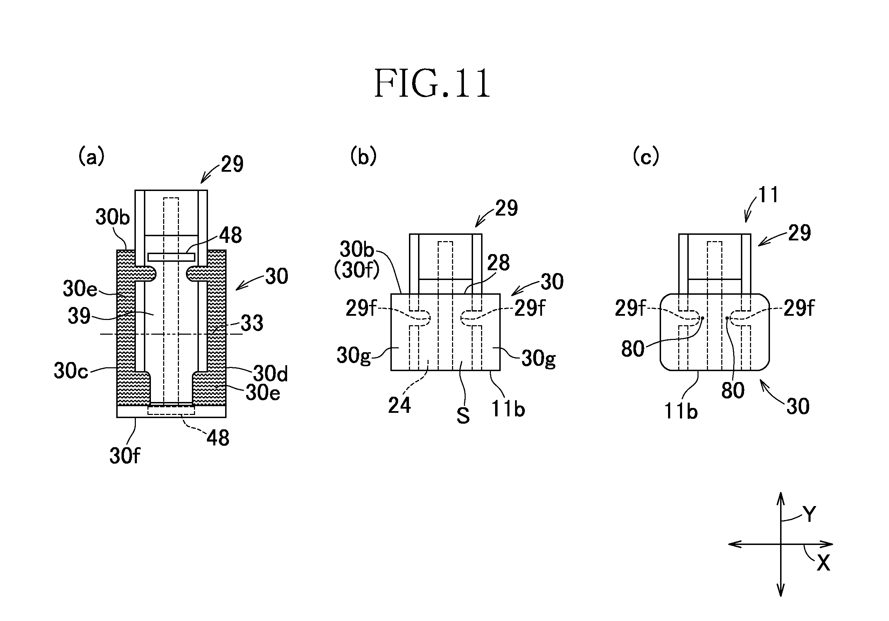

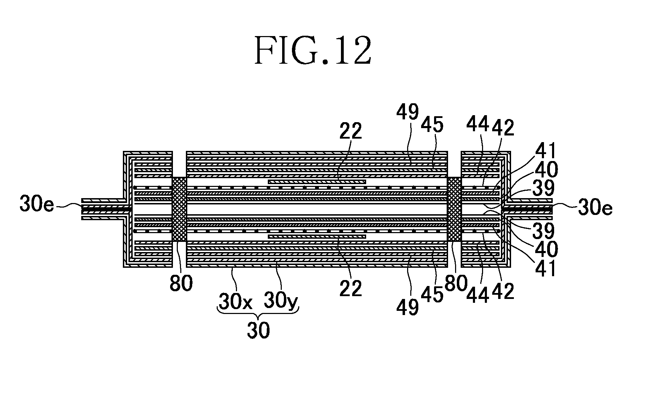

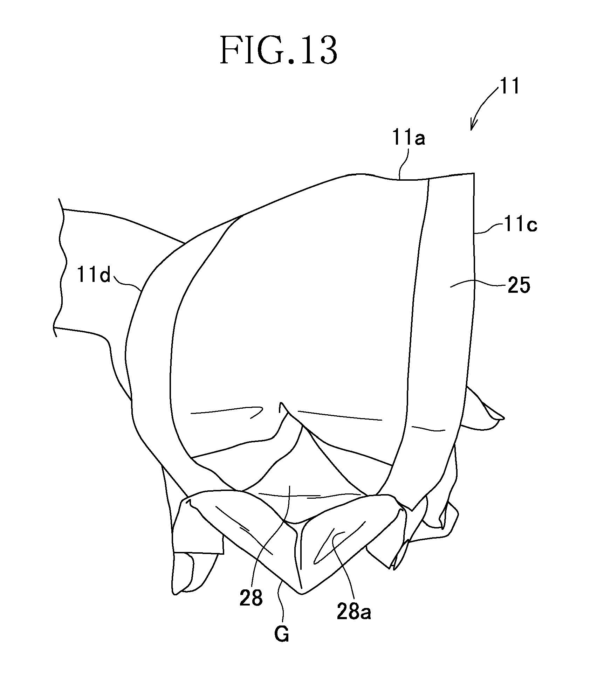

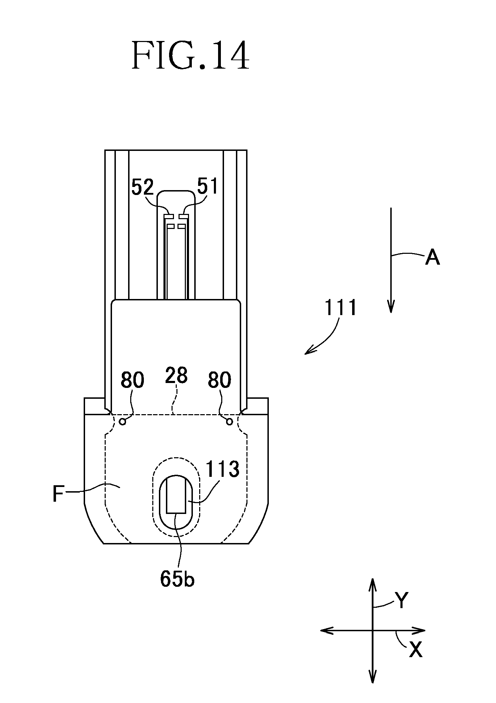

{Fig. 1} Fig. 1 is an overall diagram schematically illustrating a urine disposal device according to the present invention, inclusive of a plan view as seen from the upper side of a urine receptacle also according to the present invention.
{Fig. 2} Fig. 2 is a plan view of the urine receptacle as seen from its lower side.
{Fig. 3} Fig. 3 is a plan view of a laminated sheet and a backsheet.
{Fig. 4} Fig. 4 is an exploded perspective view of the urine receptacle.
{Fig. 5} Fig. 5 is a plan view of a urine sensor as seen from its upper side.
{Fig. 6} Fig. 6 is a perspective view of a urine collecting container.
{Fig. 7} Fig. 7 is a sectional view taken along line VII-VII in Fig. 1.
{Fig. 8} Fig. 8 is a view similar to Fig. 7, illustrating the urine receptacle put on a wearer's genitalia.
{Fig. 9} Fig. 9 is a diagram illustrating a position of a urine collecting container relative to the urine receptacle.
{Fig.10} Fig. 10 (a) throughFig. 10 (c) are diagrams illustrating the steps of assembling the urine receptacle.
{Fig.11} Fig. 11 (a) throughFig. 11 (c) are diagrams illustrating the steps of assembling the urine receptacle.
{Fig. 12} Fig. 12 is a sectional view taken along line XII-XII in Fig. 1.
{Fig. 13} Fig. 13 is a diagram illustrating the urine receptacle with an opening in an opened state when the urine receptacle is put on the wearer's genitalia.
{Fig. 14} Fig. 14 is a partially scale-enlarged view of the urine receptacle according to another embodiment of the present invention.

### {Description of Embodiments}

Fig. 1 is the overall diagram schematically illustrating the urine disposal device according to the present invention and Fig. 2 is the plan view of the urine receptacle as seen from its lower side. Referring to Figs. 1 and 2, X indicates a transverse direction and Y indicates a longitudinal direction being orthogonal to the transverse direction X. Referring also to Figs. 1 and 2, P indicates an imaginary longitudinal center line bisecting a generally bag-shaped urine receptacle 11 in the transverse direction X and Q indicates an imaginary transverse center line bisecting the urine receptacle 11 in the longitudinal direction Y.

As illustrated in Fig. 1, a urine disposal device 10 includes the urine receptacle 11, a connector 12, a urine tank 14 and a vacuum pump 15.

The urine receptacle 11 is dimensioned so that a length dimension in the longitudinal direction Y is longer than a length dimension in the transverse direction X and disposed with a urine collecting container 13. The urine collecting container 13 of the urine receptacle 11 is connected to the urine tank 14 through the connector 12. The connector 12 is adapted also to connect the urine receptacle 11 to external equipment O as described later and includes a first connecting tube 12a and a joint 12b. The urine tank 14 is connected to the vacuum pump 15 through a second connecting tube 12b.

A control unit 17 is connected to the vacuum pump 15 through an electric wire 16 and an electric connector 20 is attached to a distal end of electric wires 18a, 18b extending from the control unit 17. The vacuum pump 15 functions to suction urine from a urine receiving space S described later.

The electric connector 20 has a first terminal (not shown) adapted for electrically connecting the electric wire 18a to a first electrode 51 arranged adjacent to an end 22a of a urine sensor 22 and a second terminal (not shown) adapted for electrically connecting the electric wire 18b to a second electrode 52 arranged adjacent to the end 22a of the urine sensor 22 so that such electric connection may be switched off as the occasion demands.

In the urine disposal device 10, the urine sensor 22 detects urine discharged into the urine receiving space S and this urine detection signal is inputted to the control unit 17. The control unit 17 activates an electric motor (not shown) disposed adjacent to the vacuum pump 15 to actuate this pump. Actuation of the vacuum pump 15 causes the amount of air within the urine tank 14 to be suctioned out from the urine tank 14. Urine having been retained within the urine receiving space S to be collected first is suctioned into the urine collecting container 13 and then transferred through the first connecting tube 12a into the urine tank 14 so as to be reserved therein.

In the description given hereunder, the constituents of the urine disposal device 10 except the urine receptacle 11 and the connector 12, such as the urine tank 14, the vacuum pump 15 as well as the motor thereof and the control unit 17 will be generically designated as the external equipment O. In addition, while the urine receptacle 11 is electrically connected to the external equipment O so far as the present embodiment is concerned, it is not essential that the urine receptacle 11 is electrically connected to the external equipment O. For example, it is also possible to provide the electric connector 20 with a small sender so that a receiver set in the control unit 17 may receive the signals transmitted from the sender and, in response to such signals, the control unit 17 may activate an electric motor of the vacuum pump 15.

The urine receptacle 11 has the longitudinal direction Y, the transverse direction X being orthogonal to the longitudinal direction Y and a thickness direction Z being orthogonal to both the longitudinal direction Y and the transverse direction X (See Figs. 1, 2 and 7), and includes a first end edge 11a and a second end edge 11b spaced apart from and facing each other in the longitudinal direction Y and lateral edges 11c, 11d spaced apart from and facing each other in the transverse direction X. The urine receptacle 11 further includes a urination range 24 and extension flaps 25 contiguously extending from the urination zone 24 in the longitudinal direction Y.

The urination region 24 and the extension flaps 25 have the same length dimension so far as the transverse direction X is concerned. The urination region 24 has a length dimension in the longitudinal direction Y corresponding to about 1/3 of the urine receptacle 11 and the extension flaps 25 have a length dimension in the longitudinal direction Y corresponding to about 2/3 of the urine receptacle 11. The urination region 24 has an opening 28 extending in the transverse direction X along a boundary line between the urination region 24 and each of the extension flaps 25. The opening 28 opens into the urine receiving space S defined within the urination region 24.

Referring to Fig. 3, the urine receptacle 11 includes a laminated sheet structure 29 and a backsheet 30. As will be described later, the laminated sheet structure 29 includes two or more sheets approximately the same in shape as well as in size layered together and has a length dimension in the longitudinal direction Y larger than in the transverse direction X. The laminated sheet structure 29 has a first end edge 29a and a second end edge 29b extending in the transverse direction X and lateral edges 29c, 2 9d extending in the longitudinal direction Y between the both end edges 29a, 29b.

The lateral edges 29c, 2 9d have rectilinear portions 29e between which the length dimension in the transverse direction X is kept constant and narrow portions 29f between which the length dimension in the transverse direction X is smaller than that between the rectilinearportions 29e. Apairof the narrow portions 2 9f are formed to face each other across the imaginary longitudinal center line Q and arranged adj acent to the opening 28 as illustrated in Figs. 1 and 2. The respective narrow portions 29f are generally shaped in arcs. As will be described later in detail, a care person inserts the fingers into these narrow portions 29f, thereby to open the opening 28. These narrow portions 29f are formed, for example, by partially cutting out the laminated sheet structure 29.

The backsheet 30 has a length dimension in the transverse direction X larger than any constituent sheet in the laminated sheet structure 29. The backsheet 30 has a first end edge 30a and a second end edge 30b extending in the transverse direction X and lateral edges 30c, 30d extending in the longitudinal direction Y between the end edges 30a, 30b.

Referring again to Fig. 3, the backsheet 30 is layered on the laminated sheet structure 29 in the thickness direction orthogonal to both the transverse direction X and the longitudinal direction Y so as to extend beyond the first end edge 29a of the laminated sheet structure 29.

The backsheet 30 is formed of a liquid-impermeable material. For example, the backsheet 30 is formed of a liquid-impermeable SMS fibrous nonwoven fabric, a spunbond fibrous nonwoven fabric, a plastic sheet of polyethylene or a laminate thereof. In the urine receptacle 11 according to the present embodiment, a plastic film 30y and an SMS fibrous nonwoven fabric 30x (See Fig. 7) are laminated together.

The backsheet 30 has a first fold line 32 extending in the transverse direction X along the first end edge 2 9a of the laminated sheet structure 29 and a second fold line 33 extending in the transverse direction X at a level between the first fold line 32 and the second end edge 30b. The backsheet 30 has lateral edges 34 on the outside in the transverse direction X of the laminated sheet structure 29. Referring to Fig. 4, an approximately semi-circular exposed hole 79 is formed through the backsheet 30.

Referring again to Fig. 4, the urine receptacle 11 includes the laminated sheet structure 29, the backsheet 30, the urine sensor 22, the urine collecting container 13, a hard-breathable sheet 46 and a pair of leakage-barrier sheets 48.

In the exploded state as illustrated in Fig. 4, the laminated sheet structure 29 includes, beginning at the top, a protection sheet 39, a liquid-permeable topsheet 40, a second sheet 41, an elastic rebound sheet 42, a cushion sheet 44, a diffusion sheet 45 and a suction backsheet 49. These sheets 39, 40, 41, 42, 44, 45, 49 are formed of thermoplastic synthetic resins as described later so as to have rectangular shapes approximately the same in shape as well as in size both in the transverse direction X and the longitudinal direction Y. Surfaces of these sheets 39, 40, 41, 42, 44, 45 and 49 to be contact with each other are distributed with a hot melt adhesive (not shown) at some interval and joined to each other through the hot melt adhesive.

The protection sheet 39 serves to protect the topsheet 40 and is formed of a thermoplastic synthetic resin.

The topsheet 40 is formed of a liquid-permeable fibrous nonwoven fabric made of a thermoplastic synthetic resin, for example, formed of an air-through nonwoven fabric having a mass per unit area in a range of about 20 to about 40 g/m².

Similar to the topsheet 40, the second sheet 41 is formed of a liquid-permeable fibrous nonwoven fabric made of a thermoplastic synthetic resin, for example, formed of an air-through nonwoven fabric having a mass per unit area in a range of about 15 to about 25 g/m².

The elastic rebound sheet 42 is formed in a net-like fashion from a thermoplastic synthetic resin having an elastically rebounding property and has a liquid-permeable property since it is formed in the net-like fashion. The elastic rebound sheet 42 is formed of, for example, an ethylene-vinyl acetate sheet having a thickness in a range of about 0.5 to about 1.0 mm. The elastic rebound sheet 42 has an appropriate elastically rebounding force to tighten up around the wearer's genitalia inserted through the opening 28 into the urination region 22 and to prevent the genitalia from being displaced and to prevent urine from leaking out through the opening 28. If the elastic rebound sheet 42 is not provided and the second sheet 41 is left in contact with the urine sensor 22, a portion of urine might be left behind absorbed by the second sheet 41 to keep the second sheet 41 in wet conditions even after a process of urine suction has been finished. When the second sheet 41 in such wet conditions comes in contact with the urine sensor 22, for example, under the bodyweight of the wearer, the urine sensor 22 might erroneously detect urination. However, the elastic rebound sheet 42 is interposed between the second sheet 41 and the urine sensor 22 for the urine receptacle according to the present embodiment, whereby it is assured to prevent such erroneous detection. In this regard, the elastic rebound sheet 42 is preferably secured to the cushion sheet 44 through partially distributed adhesive to the cushion sheet 44 to assure the liquid-permeability of the cushion sheet 44.

The cushion sheet 44 is formed of a liquid-permeable nonwoven fabric sheet such as a thermal-bond nonwoven fabric sheet or an SMS nonwoven fabric sheet formed from a thermoplastic synthetic resin. The cushion sheet 44 has, for example, a mass per unit area in a range of about 20 to about 30 g/m². The cushion sheet 44 functions to prevent a portion of urine which might be present in the diffusion sheet 45, the suction backsheet 49 and the hard-breathable sheet 46 from flowing toward the electrodes 51, 52.

The diffusion sheet 45 is formed primarily from rayon fibers formed of a thermoplastic synthetic resin and hydrophilic fibers formed from a thermoplastic synthetic resin is mixed therewith to assure a liquid-permeability. The diffusion sheet 45 is used for the purpose of, as soon as urine is discharged, diffusing such urine in the transverse direction X as well as in the longitudinal direction Y, thereby rendering the suction backsheet 49 in wet conditions over a wide range. When the suction backsheet 49 becomes wet, an internal space 64 of the urine collecting container 13 creates a negative pressure which, in turn, facilitates urine to be suctioned into the internal space 64 of the urine collecting container 13.

The suction backsheet 49 is formed of a thermoplastic synthetic resin. The suction backsheet 49 has a mass per unit area in a range of about 70 to about 80 g/m² and, as material thereof, an airlaid fibrous nonwoven fabric maybe used. The suction backsheet 49 has the function and the property similar to the hard-breathable sheet 46 and may be formed of a similar material.

Referring to Fig. 5, the urine sensor 22 includes an insulating base sheet 50 formed of an approximately rectangular plastic film, the first electrode 51 and the second electrode 52 printed on the upper surface of the insulating base sheet 50 with an electrically conductive material such as electrically conductive ink or electrically conductive paint. The insulating base sheet 50 may be formed of, for example, a polyester film having a dimension in the thickness direction Z in a range of about 50 to about 100 µm. The electrodes 51, 52 are printed with ink containing artificial graphite such as carbon black in a range of about 3 to about 7 % by mass or carbon graphite in a range of about 10 to about 30 % by mass to make these electrodes 51, 52 electrically conductive. The insulating base sheet 50 is formed in a central region with a longitudinally long rectangular opening 53. The first and second electrodes 51, 52 are spaced by this opening 53 from each other so as to face each other and extending in the longitudinal direction Y. The respective electrodes 51, 52 have a plurality of urine detecting regions 54 exposed from the insulating base sheet 50. In the urine sensor 22 of such arrangement, voltage is applied between the electrodes 51, 52 and urination is detected on the basis of a current differential measured in ordinary state and in a state in which urine has been discharged into the urine receiving space S of the urine receptacle 11.

Referring to Fig. 6, the urine collecting container 13 may be formed of, for example, a flexibly elastic and liquid-impermeable thermoplastic synthetic resin such as soft polyethylene or silicon rubber. The urine collecting container 13 has a flexibility allowing the container to be curved in the longitudinal direction Y and in the transverse direction X but has a stiffness enough to withstand a remarkable deformation owing to a negative pressure generated when the vacuum pump suctions urine. The urine collecting container 13 includes a bottom 60, a peripheral wall 61 rising from the bottom 60, an upper opening 62 surrounded by the peripheral wall 61 and a flange 63 extending outward from a top edge of the peripheral wall 61. Within the urine collecting container 13, the internal space 64 is defined by the suction backsheet 49 arranged so as to cover the bottom 60, the peripheral wall 61 and the upper opening 62.

The urine collecting container 13 is disposed with a tubular member 65 extending in the longitudinal direction Y across an approximately central region. The tubular member 65 has an internal outlet port 65a arranged within the internal space 64 of the urine collecting container 13 and an external output 65b arranged at the outside of the internal space 64 so that the internal outlet port 65a and the external outlet port 65b may be in a fluid-communication relationship. To the external output 65b, the first connecting tube 12a is attached through the joint 12c (See Fig. 1). In other words, the external outlet port 65b of the urine connecting container 13 functions as a joining section. The urine collecting container 13 evacuates urine within the internal space through the internal outlet port 65a of the tubular member 65 in the urine evacuation direction A to the external outlet port 65b until urine within the internal space 64 becomes empty.

In the urine collecting container 13, a plurality of convexities projecting from the bottom is formedonbothsides in the transverse direction X of the tubular member 65. In addition, a plurality of convexities 67 projecting towards the internal outlet port 65a is formed on a portion of the peripheral wall 61 facing the internal outlet port 65a.

Referring to Fig. 7, the urine collecting container 13 is arranged in the urine receptacle 11 in such a manner that the urine evacuation direction A extending from the internal outlet port 65a to the external outlet port 65b is oriented toward the opening 28.

The internal space 64 of the urine collecting container 13 has a length dimension in the longitudinal direction Y which is smaller than 1/2 of a length dimension in the longitudinal direction Y of the urination region 24. The urine collecting container 13 is arranged adjacent to the second end edge 11b of the urine receptacle 1 so that the internal space 64 of the urine collecting container 13 is positioned in a lower half in the longitudinal direction Y of the urination region 24.

Fig. 8 is a sectional view illustrating the urine receptacle 11 put on the wearer. In a state that the urine receptacle 11 is put on the wearer in the supine posture, the urine receptacle 11 has a first surface G facing the wearer's skin and a second surface F not facing the wearer's skin. As illustrated, the urine collecting container 13 is arranged on the second surface of the urine receptacle 11.

Fig. 9 is a diagram illustrating an arrangement of the urine collecting container 13. High stiffness regions 80 as described in more detail later are paired so as to be symmetric about the imaginary longitudinal center line Q. Each of the high stiffness regions 80 is formed in a circular shape and has a center. In this regard, the urine collecting container 13 is illustrated in Fig. 9 so that the internal space 64 thereof may be exposed.

A region defined under a first imaginary line M1 passing through the centers of the respective high stiffness regions 80, positioned on one side in the transverse direction X and being tangent to the internal space 64 on the upper side of the urine collecting container 13, and a region defined under a second imaginary line M2 passing through the center of the high stiff region 80, positioned on the other side in the transverse direction X and being tangent to the internal space 64 on the upper side of the urine collecting container 13, overlap each other to define an overlapping region 90. The urine collecting container 13 is arranged in the urine receptacle 11 so that the internal space 64 may be positioned in this overlapping region 90. More specifically, in a state that the imaginary longitudinal center line Q extends in parallel to vertical lines M3, M4 passing through the centers of the respective high stiffness regions 80 positioned on respective sides in the transverse direction X, the internal space 64 of the urine collecting container 13 is arranged within the overlapping region 90 defined so that an angle θ1 formed between the vertical line M3 passing through the center of the high stiffness region 80 on one side in the transverse direction X is 45° or less and an angle θ2 formed between the vertical line M4 passing through the center of the high stiffness region 80 on the other side in the transverse direction X and the second imaginary line M2 is 45° or less. In this regard, the urine collecting container 13 is arranged in the illustrated embodiment so that the upper side of the internal space 64 may be tangent to the imaginary lines M1, M2 extending at angles θ1, θ2 of about 25°.

Referring again to Fig. 4, the hard-breathable sheet 46 is formed at a center thereof with a hole 57 adapted to be aligned with the upper opening 62 of the urine collecting container 13. The hard-breathable sheet 46 is joined to the urine collecting container 13 through adhesive distributed to the flange 63. The hard-breathable sheet 46 is liquid-permeable but approximately or absolutely hard-breathable and formed from a thermoplastic synthetic resin. As material for the hard-breathable sheet 46, for example, an SMS nonwoven fabric without any treatment or treated to become hydrophilic with use of surface acting agent may be used. A breathability of the hard-breathable sheet 46 is preferably in a range of 0 to 100 cc/cm²/sec in wet conditions and in a range of 20 to 200 cc/cm²/sec.

The leakage-barrier sheet 48 is formed from a laminate of two or more appropriately rectangular sheets formed from a thermoplastic synthetic resin. For example, the leakage-barrier sheet 48 may be formed of 4 to 8 air-through nonwoven fabric sheets having a mass per unit area in a range of about 30 to about 40 g/m² laminated in the thickness direction Z. The leakage-barrier sheet 48 is arranged along the periphery of the opening 28.

Now the steps of assembling the urine receptacle 11 arranged as has been described hereinbefore will be described.

First, predetermined portions are cut away from the laminated sheet structure 29 illustrated in Fig. 10 (a). As a result of the cutting step, along the lateral edges 29c, 29d extending in the longitudinal direction Y between the end edges 29a, 29b, there are the rectilinear portions 29e and the narrow portions 2 9f having a length dimension smaller than that of the rectilinear portions 29e. On the side of the first end edge 29a, there is a distal end portion 2 9g having a length dimension in the transverse direction X smaller than that of the rectilinear portions 29e.

The narrow portions 29f are arranged adjacent to the opening 28 as will be described later (See Fig. 2). The narrow portions 29f are arrangements into which the wearer's or the care person's fingers will be inserted to open the opening 28. Differential dimensions in the thickness direction between the laminated sheet structure 29 including seven sheets 39, 40, 41, 42, 44, 45, 49 and the backsheet 30 enables the wearer or the care person to recognize these narrow portions 29f by tactual sense. It is also possible for the wearer or the care person to recognize visually the narrow portions 29f so long as a total light transmittance of the backsheet 30 is in a range of about 30 to about 70 %. In the urine receptacle 11 according to the present embodiment, the backsheet 30 having the total light transmittance of about 40 % is used.

After the hard-breathable sheet 46 has been attached to the urine collecting container 13 with the use of adhesive, the urine collecting container 13 is arranged on the backsheet 30 so that the external outlet port 65b of the urine collecting container 13 may be exposed through an exposing hole 79 and, as illustrated in Fig. 10 (b), the urine collecting container 13 is attached to the backsheet 30 with the hot melt adhesive distributed to the backsheet 30 through the hard-breathable sheet 46. Then, all over the upper surface of the backsheet 30 is formed with adhesive regions 30e with the use of adhesive such as hot melt adhesive.

Referring now to Fig. 10 (c), the backsheet 30 is joined to the laminated sheet structure 29 through the adhesive regions 30e. In the state of the backsheet 30 and the laminated sheet structure 29 having been laminated together, a pair of the leakage-barrier sheets 48 are attached to the upper surface of the protection sheet 39. In Fig. 10 (c), the lower side leakage-barrier sheet 48 is arranged adjacent to the first end edge 29a of the laminated sheet structure 29 and the upper side leakage-barrier sheet 48 is arranged adjacent to the second end edge 30b of the backsheet (30).

Then, the backsheet 30 is folded along the first fold line 32 so as to cover the lower side leakage-barrier sheet 48 and, as illustrated in Fig. 11 (a), the portions of the backsheet 30 facing each other are joined together. In the state folded along the first fold line 32, the lower end of the backsheet 30 defines a third end edge 30f and the second fold line 33 coincides with a line bisecting the second end edge 30b and the third end edge 30f.

Now, the laminated sheet structure 29 and the backsheet 30 are folded along the second fold line 33 of the backsheet 30. More specifically, the laminated sheet structure 29 and the backsheet 30 are folded so that the protection sheet 39 may face itself and be put in contact with itself, whereby the third end edge 30f and the second end edge 30b of the backsheet 30 are put in coincidence with each other as illustrated in Fig. 11 (b). The lateral edges 30c, 30d of the backsheet 30 are joined to each other to form joined lateral edge portions 30g on both sides in the transverse direction X and the opening 28 is formed in the thickness direction Z between the third end edge 30f and the second end edge 30b. In this way, the urine receiving space S in fluid communication with this opening 28 is formed within the urination region 24. The second fold line 33 defines the second end edge 11b of the urine receptacle 11.

After four corners of the backsheet 30 have been cut off, the sheets 39, 40, 41, 42, 44, 45 and 49 are heat sealed together in the thickness direction to form the high stiffness regions 80, thereby obtaining the urine receptacle 11 as illustrated in Fig. 11 (c).

Referring to Fig. 12, each of the high stiffness regions 80 includes the thermoplastic synthetic resin sheets 39, 40, 41, 42, 44, 45 and 49 integrally heat sealed in the thickness direction Z so as to be continuous from the uppermost surface to the bottom surface in the thickness direction Z of the urine receptacle 11. The sheets 39, 40, 41, 42, 44, 45 and 49 are integrally sealed together to form the high stiffness regions 80 and necessarily stiffness higher than that in the other regions is assured by the high stiffness regions 80.

Referring again to Fig. 2, the high stiffness regions 80 are formed so as to be on an imaginary shortest line 85 indicating the smallest length dimension in the transverse direction X of the laminated sheet structure 29 defined between a pair of the narrow portions 29f.

Each of the high stiffness regions 80 may be formed as a circle having a diameter of 4 mm so as not to be put in contact with the narrow portion 29f.

The adhesive regions 30e are formed of the adhesive distributed over the whole area on the upper surface of the backsheet 30 and exist also adjacent to the respective narrow portions 29f. In consequence, the backsheet 30 is joined to itself adjacent to the respective narrow portions 29f through the adhesive regions 30e and joined lateral edge portions 30g extending in the longitudinal direction Y are formed on both sides in the transverse direction X of the urination region 24.

Since the high stiffness regions 80 are arranged as has been described above, the respective joined lateral edge portions 30g have extensions extending in the longitudinal direction Y closer to the opening 28 than to the high stiffness regions 80. A distance dimension L1 between a pair of the high stiffness regions 80 in the transverse direction X is shorter than a length dimension L2 in the transverse direction X of the opening 28 (See Fig. 2).

When it is desired to put the urine receptacle 11 on the genitalia, for example, the wearer's thumb is inserted into one of the narrow portions 29f and the wearer's first finger into the other narrow portion 29f.

Then the thumb and first finger may be moved closer to each other to assure that the mid portions in the transverse direction X of the respective sheets 39,40, 41, 42, 44, 45 and 49 having a relatively high bending moment are folded and the opening 28 is smoothly opened.

Now the forefront of the genitalia may be inserted into the opening 28 and further inserted toward the back of the urination region 24. The distance dimension L1 (See Fig. 2) in the transverse direction X between a pair of the high stiffness regions 80 is shorter than the length dimension L2 in the transverse direction X of the opening 28 and the high stiffness regions 80 are arranged rather within the urine receiving space S with respect to the opening 28. Consequently, the high stiffness regions 80 function as guide means for the forefront of the genitalia.

Upon insertion of the genitalia to the back of the urination region 24, the thumb and the first finger may be disengaged from the urine receptacle 11. Thereupon, the opening 28 is closed under the elastic rebounding force of the elastic rebound sheet 42. In this manner, as illustrated in Fig. 8, it is possible to put the urine receptacle 11 on the genitalia R without touching the genitalia R with the wearer's hand.

The high stiffness regions 80 are arranged closer to the imaginary longitudinal center line Q than the joined lateral edge portions 30g are. Consequently, pubic hair should not be caught by the adhesive regions 30e during insertion of the genitalia R.

A length dimension L3 in the longitudinal direction Y of the urination region 24 is preferably in a range of 50 to 180 mm and, more preferably, in a range of 80 to 130 mm.

The urination region 24 is the region adapted to receive the genitalia R inserted thereinto and to receive urine. In view of this, while it is rather preferable to determine the length dimension L3 in the longitudinal direction Y of the urination region 24 to be relatively long, if the dimension L3 is excessively long, the lower end of the urine receptacle might come in contact with a wheel chair on the assumption that the wearer is in the wheel chair when he urinates. In contrast, if the dimension is excessively short, it maybe difficult to stably hold the genitalia within the urine receptacle. From this viewpoint, preferably an average length value of genitalia of Japanese men is calculated and the dimension L3 is determined in consideration of the calculated average value.

The distance dimension L1 in the transverse direction X between a pair of the high stiffness regions 80 is a factor substantially specifying the genitalia R which can be properly inserted into the urine receptacle 11. Considering the average genitalia thickness of Japanese men, the distance dimension L1 of 50 mm or more is sufficient to assure the desired function. Taking account of erection, the distance dimension L1 is preferably 75 mm or more. If the distance dimension L1 is excessively large, the elastically rebounding force of the elastic rebound sheet 42 might be insufficient to close the opening 28 and a portion of urine discharged into the urine receptacle might leak out through the opening. Specifically, the distance dimension L1 of 130 mm or more is inappropriate. From these viewpoints, the distance dimension L1 is preferably in a range of 50 to 130 mm and more preferably in a range of 75 to 130 mm.

The length dimension L2 in the transverse direction X of the urination region 24 is preferably in a range of about 60 to about 170 mm and more preferably in a range of about 85 to about 140 mm.

From the viewpoint of urine retention capacity, it will be preferable to determine the length dimension L2 in the transverse direction X of the urination region 24 to be relatively large. However, when the length dimension L2 is excessively large, the lateral portions of the urine receptacle 11 might be put in contact with the wearer's inner thighs. In contrast, if the length dimension L2 is excessively small, it will be difficult to hold the genitalia R stably. Considering these undesirable cases, the length dimension L2 is preferably within the range as described above.

The length dimension L4 in the transverse direction X of the urine receptacle 11 is a sum of the length dimension L2 in the transverse direction X of the urination region 24 and a length dimension two times the total length dimension L5 in the transverse direction X of a pair of the adhesive regions 30e of the backsheet 30.

In the urine disposal device 10 according to the present embodiment put on the wearer in supine posture, the external outlet port (connecting region) 65b of the urine receptacle 11 is arranged on the second surface F not facing the wearer's skin. In consequence, when the wearer discharges excrement, the skin-contact surface of the urine receptacle 11 covers the first connecting tube 12a and the external outlet port 65b, whereby the distal end portion of the first connecting tube 12a and the external outlet port 65b are unlikely to be soiled with the excrement. In addition, the external outlet port 65b is unlikely to come in contact with the wearer's body since the external outlet port 65b is arranged on the second surface F. In this way, it is possible to alleviate a sense of discomfort felt by the wearer even if the urine collecting container 13 is formed of a thermoplastic synthetic resin.

In the state that the urine receptacle 11 is arranged so that the imaginary longitudinal center line Q extends in parallel to the vertical lines M3, M4, the urine collecting container 13 is arranged so that the internal space 64 may be arranged within the overlapping region 90 and the internal space 64 of the urine collecting container 13 is arranged within the overlapping region 90 defined so that an angle θ1 formed between the vertical line M3 passing through the center of the high stiffness region 80 on one side in the transverse direction X is 45° or less and an angle θ2 formed between the vertical line M4 passing through the center of the high stiffness region 80 on the other side in the transverse direction X and the second imaginary line M2 is 45° or less. Such arrangement is effective to prevent urine from leaking out through the opening 28 even when the wearer of the urine receptacle 11 is in a lying posture. Furthermore, the urine collecting container 13 is arranged so that the internal space 64 occupies a lower half of the urination region 24 and such arrangement assures that the discharged urine is reliably suctioned.

Furthermore, the urine collecting container 13 is arranged in the urine receptacle 11 in such a manner that the urine evacuation direction A is oriented toward the opening 28. Such arrangement of the urine collecting container 13 makes it possible to arrange the internal outlet port 65 adjacent to the second end edge 11b of the urine receptacle 11, whereby urine is unlikely to remain in the urine receiving space S.

In addition, the high stiffness regions 80 are locally formed as a portion of the periphery 28a of the opening 28 in the course of heat sealing a plurality of sheets 39, 40, 41, 42, 44, 45 and 49 and, more specifically, these high stiffness regions 80 are formed only on the respective lateral edges 30c, 30d in the transverse direction X of the sheets 39, 40, 41, 42, 44, 45 and 49. Consequently, the stiffness in the middle portions in the transverse direction X of these sheets is relatively low and the sheets 39, 40, 41, 42, 44, 45 and 46 are easily bent. In this manner, it is possible to provide the urine receptacle 11 of which the opening 28 may be smoothly opened.

Now functional behaviors of the urine receptacle 11 and the urine disposal device 10 using this urine receptacle 11 will be described. Urine discharged in the urination region 24 of the urine receptacle 11 is temporarily retained within the urine receiving space S defined within the urine receptacle 11. The amount of urine temporarily retained within the urine receiving space S permeates the topsheet 40, the second sheet 41 and the elastic rebound sheet 42 and come in contact with the urine sensor 22. When any amount of urine bridges the electrodes 51, 52 of the urine sensor 22, the urine detection regions 54 detect the presence of urine.

Based on detection by the urine sensor 22, the control unit 17 actuates the vacuum pump 15. In response of actuation of the vacuum pump 15, the urine tank 14 enters a vacuum state and urine which has been retained within the internal space 64 of the urine collecting container 13 is suctioned through the first connecting tube 12a into the urine tank 14 and the urine receiving space S is evacuated. When the urine sensor 22 becomes unable to detect any amount of urine, the control unit 17 ceases to drive the vacuum pump 15 on the basis of non-detection by the urine sensor 22.

The kinds of material for the constituent members of the urine receptacle 11 according to the present invention are not limited to those described in this specification but the other various types of material widely used in the relevant technical field may be used without limitation. The terms "first", "second" and "third" used in the specification and Claims of the present invention are used merely to distinguish similar elements or similar positions.

For the laminated sheet structure 29, it is not essential to use all of the sheets 39, 40, 41, 42, 44, 45 and 49 but it is possible for any purpose to omit one or more from these sheets 39, 40, 41, 42, 44, 45 and 49. It is also possible to add any other kinds of sheets to these sheets as necessary.

While the embodiment according to which the adhesive regions 30e are formed over the entire upper surface of the backsheet 30 has been described, it is not essential to form such adhesive regions 30e over the entire area but it is also possible to form the adhesive regions 30e only in regions requiring them and to leave non-adhesive regions.

While the embodiment according to which only the protection sheet 39 is provided for the purpose of protecting the topsheet 40 only has been described, it is possible to provide also a protection sheet to protect the leakage-barrier sheet 48.

The urine receptacle 11 may be assembled by following other steps of assembling different from the steps of assembling as has been described above.

While the embodiment according to which the leakage-barrier sheets 48 are not included in the sheets which are heat sealed together to form the high stiffness regions 80 has been described, the sheets including also the leakage-barrier sheets 48 may be heat sealed together to improve stiffness of the high stiffness regions 80, whereby the opening 28 may be more smoothly opened.

While the stiffness regions 80 have been described to have the circular shapes, the shape of the high stiffness regions 80 may be arbitrarily selected and, for example, the stiffness regions 80 may have polygonal shapes.

Now the urine receptacle 111 in the urine disposal device according to another embodiment of the present invention will be described hereunder. In this regard, the same constituents of the urine receptacle 11 as those in the embodiment described above are designated by the same reference numerals or signs to avoid repetitive explanation.

In the urine receptacle 111 according to this embodiment, the urine collecting container 113 is arranged within the urine receptacle 111 so that the urine evacuation direction A from the internal outlet port 65a to the external outlet port 65b is not oriented to the opening 28 but reversely oriented. The urine collecting container 113 is arranged on the second surface F of the urine receptacle 111 for the wearer in a supine posture.

In this urine receptacle 111, the urine collecting container 113 is arranged within the urine receptacle so that the urine evacuation direction A may be not oriented toward the opening but reversely oriented and consequently it is possible to arrange the external outlet port 65b functioning also as the connecting region adj acent to the second end edge 11b of the urine receptacle 111, thereby facilitating the connector 12 to be connected with the external outlet port 65b.

### {Reference Signs List}

- 10: urine disposal device
- 11: urine receptacle
- 12: connector
- 13: urine collecting container
- 15: vacuum pump
- 24: urination range
- 28: opening
- 29: laminated sheet structure
- 39: protection sheet (sheet)
- 40: topsheet (sheet)
- 41: second sheet (sheet)
- 42: elastic rebound sheet (sheet)
- 44: cushion sheet (sheet)
- 45: diffusion sheet (sheet)
- 49: suction back sheet (sheet)
- 65: tubular member
- 65a: interior outlet
- 65b: exterior outlet (connecting region)
- 80: high stiffness regions
- 111: urine receptacle
- 113: urine collecting container
- A: urine discharging direction
- F: second surface (non-skin-facing surface)
- G: first surface (skin facing surface)
- O: external equipment
- S: urine receiving space
- X: transverse direction
- Y: longitudinal direction
- Z: thickness direction

## Claims

1. A urine disposal device having a longitudinal direction, a transverse direction and a thickness direction and including a urine receptacle defining a urine receiving space and adapted to connect the urine receptacle through a connector to external equipment at least including a vacuum pump for suction of urine from the urine receiving space, wherein:
the urine receptacle has a first surface facing a wearer's skin, a second surface not facing the wearer's skin when the wearer is in a supine posture and a connecting region in which the connector is connected; and
the connecting region is arranged on the second surface.

2. The urine disposal device according to Claim 1, wherein:
the urine receptacle is disposed with a urine collecting container having an internal space;
the urine collecting container includes an internal outlet port arranged within the internal space, an external outlet port arranged on an outside of the internal space and a tubular member adapted to put the internal outlet port and the external outlet port in a fluid-communication relationship; and
the external outlet port functions also as a connecting region.

3. The urine disposal device according to Claim 2, wherein:
the urine receptacle has an opening extending in the transverse direction; and
the urine collecting container is arranged so that a urine evacuation direction from the internal outlet port to the external outlet port is oriented oppositely to the opening.

4. The urine disposal device according to Claim 2, wherein:
the urine receptacle has an opening extending in the transverse direction; and
the urine collecting container is arranged so that the urine evacuation direction from the internal outlet port to the external outlet port is oriented toward the opening.

5. The urine disposal device according to any one of Claims 1 through 4, wherein:
the urine receptacle is formed of a plurality of sheets formed from a thermoplastic synthetic resin in a bag-like shape defining therein a urine receiving space in fluid-communication with the opening; and
high stiffness regions are formed in the course of heat sealing a plurality of sheets but the high stiffness regions are formed only on lateral edges of these sheets as a portion of the opening's periphery.

6. The urine disposal device according to Claim 5, wherein:
the respective high stiffness regions have centers; and
the urine collecting container is arranged so that the internal space is positioned within an overlapping region in which a region defined under a first imaginary line extending so as to pass through the center of one of two high stiffness regions spaced apart from each other in the transverse direction and to be tangent to the internal space on the upper side of the urine collecting container and a region defined under a second imaginary line extending so as to pass through the center of the other high stiffness region and to be tangent to the internal space on the upper side of the urine collecting container overlap each other.
